# EUROPÄISCHE PATENTSCHRIFT

(11) **EP 1 324 764 B1**
(45) Veröffentlichungstag und Bekanntmachung des Hinweises auf die Patenterteilung: **27.07.2005**
(21) Anmeldenummer: 01986263.0
(22) Anmeldetag: 05.10.2001
(51) Int. Cl.: A61K 38/09, A61D 19/00

(54) **VERFAHREN ZUM ZYKLUSSTART BEI WEIBLICHEN ZUCHTTIEREN**
METHOD FOR INDUCING ONSET OF THE REPRODUCTIVE CYCLE OF FEMALE BREEDING ANIMALS
PROCEDE POUR INDUIRE LE DEBUT DU CYCLE CHEZ DES ANIMAUX D'ELEVAGE DE SEXE FEMININ

(30) Priorität: 05.10.2000 DE 10050831
(43) Veröffentlichungstag der Anmeldung: 09.07.2003
(73) Patentinhaber: Veyx-Pharma GmbH, 34639 Schwarzenborn (DE)
(72) Erfinder: ZAREMBA, Wolfgang, 34576 Homberg (DE); HÜHN, Uwe, 98617 Wölfershausen (DE); HESS, Armin, 34639 Schwarzenborn (DE)
(74) Vertreter: Gross, Felix, Dr.
(86) Internationale Anmeldenummer: PCT/DE2001/003870
(87) Internationale Veröffentlichungsnummer: WO 2002/028405

(56) Entgegenhaltungen:
- WO-A-00/78335
- US-A- 4 753 928
- SOWER S A ET AL: "PRIMARY STRUCTURE AND BIOLOGICAL ACTIVITY OF A THIRD GONADOTROPIN- RELEASING HORMONE FROM LAMPREY BRAIN" ENDOCRINOLOGY, BALTIMORE, MD, US, Bd. 132, Nr. 3, 1993, Seiten 1125-1131, XP002067663 ISSN: 0013-7227
- YU WEN H ET AL: "A hypothalamic follicle-stimulating hormone-releasing decapeptide in the rat" PROCEEDINGS OF THE NATIONAL ACADEMY OF SCIENCES OF USA, NATIONAL ACADEMY OF SCIENCE. WASHINGTON, US, Bd. 94, Nr. 17, 1997, Seiten 9499-9503, XP002170294 ISSN: 0027-8424
- CLARK B A ET AL: "Manipulating patterns of ovarian follicle development in cattle with progesterone and gonadotrophin releasing hormone to produce oestrous cycles with two or three follicle waves." PROCEEDINGS OF THE NEW ZEALAND SOCIETY OF ANIMAL PRODUCTION, Bd. 58, 1998, Seiten 85-87, XP001145710 ISSN: 0370-2731
- LYNCH P R ET AL: "Treating cattle with progesterone as well as a GnRH analogue affects oestrous cycle length and fertility." ANIMAL REPRODUCTION SCIENCE, Bd. 56, Nr. 3-4, 16. August 1999 (1999-08-16), Seiten 189-200, XP002232439 ISSN: 0378-4320
- SCHAEFER SABINE ET AL: "Synchronisation of oestrus, induction of ovulation and batch farrowing of sows." TIERAERZTLICHE UMSCHAU, Bd. 54, Nr. 1, 1. Januar 1999 (1999-01-01), Seiten 33-36, 38, XP009006431 ISSN: 0049-3864
- PURSLEY J R ET AL: "Pregnancy rates per artificial insemination for cows and heifers inseminated at a synchronized ovulation or synchronized estrus." JOURNAL OF DAIRY SCIENCE, Bd. 80, Nr. 2, 1997, Seiten 295-300, XP009006475 ISSN: 0022-0302
- LÖSCHER W., UNGEMACH F. R., KROKER R.: "Grundlagen der Pharmakotherapie bei Haus- und Nutztieren" 1991 , VERLAG PAUL PAREY , BERLIN, HAMBURG XP002232441 Seite 297, Spalte 2, Zeile 1 -Seite 298, Spalte 1, Zeile 27
- "Cystorelin details" MERIAL PRODUKTINFORMATION CYSTORELIN, [Online] XP002232440 Gefunden im Internet: <URL:http://us.merial.com/producers/dairy/ products/dairy_cystorelin.asp#> [gefunden am 2003-02-25]
- PLONAIT H.; BICKHARDT K.: 'Lehrbuch der Schweinekrankheiten', 1988, VERLAG PAUL PAREY, BERLIN, HAMBURG

## Beschreibung

Die Erfindung betrifft ein Verfahren zum Zyklusstart bei weiblichen Zuchttieren.

Im Betriebs- und Erwerbszweig der Zuchttierhaltung und bei der Ferkelerzeugung wird zunehmend die Gruppenabferkelung praktiziert. In Verbindung mit der breiten Nutzanwendung der künstlichen Besamung stellt sie die unerlässliche Voraussetzung dafür dar, um größere Ferkelpartien in marktkonformer Qualität und mit einheitlichem Gesundheitszustand bereitstellen zu können.

Zugleich wächst das Interesse an geeigneten biotechnischen Verfahren, um die individuellen Sexualzyklen innerhalb der zusammengestellten Tiergruppen steuern zu können.

Die direkt am Fortpflanzungsgeschehen beteiligten Hormonsysteme arbeiten in einem großen Regelkreis neuroendokriner Integration. Dieser schafft die hormonellen Voraussetzungen für den Ablauf des Brunstzyklus. Natürlicherweise folgt nach dem Ende der sexuellen Aktivität, z. B. nach Ablauf des Diöstrus bei zyklischen Tieren, nach dem Absetzen und der Trennung der Muttertiere von den Jungtieren bzw. nach vorheriger Zyklusblockade vor allem unter dem Einfluss des follikelstimulierenden Hormons die Einleitung des Follikelwachstums an den Ovarien, gefolgt von Follikelreifung, Brunsteintritt, Ovulation und Befruchtungschance im Falle einer termingerechten Besamung mit befruchtungsfähigem Sperma. Die individuellen Zyklen unterliegen einer beträchtlichen biologischen Schwankungsbreite. In der Tierhaltung, insbesondere in Ferkelerzeugerbetrieben mit periodenweisem Abferkelsystem besteht aus tiergesundheitlichen und wirtschaftlichen Gründen ein Interesse an eng begrenzten Zeitspannen für die wichtigsten Fortpflanzungsereignisse, nämlich Besamung und Geburt. Die gezielte Beeinflussung des Follikelwachstums und der nachfolgenden Stadien bei Gruppen von Tieren, z. B. abgesetzten Alttieren oder zuchtreifen Tieren dient der terminlichen Ausrichtung biologischer Prozesse auf notwendige und wünschenswerte Abläufe in der Tierproduktion.

Verfahren zur Zyklussynchronisation und zur Beeinflussung der Reproduktionsfunktionen sind in den letzten Jahren in vielfältiger Form entwickelt worden. Sie basieren auf den pharmakologischen Möglichkeiten einer biotechnischen Fortpflanzungssteuerung.

Die entwickelten Verfahren in der Ferkelzucht zielen auf die Beeinflussung des Zyklusstartes, sowie die Brunst- und Ovulationssynchronisation ab (H.Plonait, K.Bickhardt, Lehrbuch der Schweinekrankheiten, 1988, Verlag Paul Parey, Berlin, Hamburg, S. 266-269)

In der Sauenhaltung ist z.B. der Brunststart durch Gabe von Hormonen bekannt. Dafür kommt bislang die zyklusgerechte Anwendung gonadotroper Hormonpräparate natürlichen Ursprungs im Anschluss an eine vorausgegangene Zyklusblockade, z. B. durch die Säugezeit bei Alttieren und die medikamentöse Brunstsynchronisation bei Jungtieren in Betracht.

Bei Jungtieren kann die Brunstsynchronisation durch eine zeitliche Verschiebung des Follikelwachstums und der Ovulation z.B. durch den Einsatz von Methallibur, welches die Wirkung des Gonadotropin-Releasing Hormons (GnRH) hemmt, erfolgen. Methallibur wurde jedoch in Westeuropa vom Markt genommen. Als Neuentwicklung wird in Westeuropa Allyl-Trenbolon, ein Steroid mit gestagener Wirkung, mit Erfolg verwendet (H.Plonait, K.Bickhardt, Lehrbuch der Schweinekrankheiten, 1988, Verlag Paul Parey, Berlin, Hamburg, S. 266-269).

Darüberhinaus ist die Anwendung des Stutenserumgonadotropins PMSG (=PMSG; oder synonym: equines Choriongonadotropin, eCG) bekannt. Es hat sich als sehr erfolgreich für die medikamentöse Brunstsynchronisation bei Altsauen nach dem Absetzen der Ferkel sowie bei Jungsauen nach einer Vorbehandlung mittels Zyklusblockade erwiesen (H.Plonait, K.Bickhardt, Lehrbuch der Schweinekrankheiten, 1988, Verlag Paul Parey, Berlin, Hamburg, S. 266-269; Schäfer et al, 1999,Tierärztliche Umschau, Bd. 54, Nr. 1). PMSG wird mittels Aderlass-Methode von tragenden Stuten gewonnen. Dagegen mehren sich tierschützerische Einwände. Ebenso kann die Anwendung bestimmter PMSG-Chargen, insbesondere solcher in flüssigkonservierter Form, zu unerwünschten Nebenwirkungen beitragen, z. B. anaphylaktischen Schockreaktionen.

Ein weiterer Eingriffspunkt in den Reproduktionsprozess ist die medikamentöse Ovulationssynchronisation, die bevorzugt durch GnRH erreicht wird (Schäfer et al, 1999,Tierärztliche Umschau, Bd. 54, Nr. 1).

Der Erfindung liegt die Aufgabe zugrunde, ein Verfahren zum Zyklusstart für Zuchttiere zu entwickeln, das dem natürlichen biologischen Ablauf des Sexualzyklus angepasst ist und in einer Zuchttiergruppe eine terminliche Steuerung des beginnenden Follikelwachstums an den Eierstöcken sowie des Brunsteintritts bewirkt.

Erfindungsgemäß wird die Aufgabe dadurch gelöst, dass beim Verfahren zum Zyklusstart bei Zuchttieren ein Mittel eingesetzt wird, das neben üblichen Hilfsstoffen als Wirkstoff synthetisch hergestellte FSH-Releasinghormone mit bekannten Sequenzen, bestehend aus 10 Aminosäuren (Dekapeptid), enthält. Das genannte Dekapeptid besitzt die Aminosäuresequenz

Pyr-His-Trp-Ser-His-Asp-Trp-Lys-Pro-Gly-NH₂. Diese Sequenz kann durch Anfügung von weiteren Bestandteilen modifiziert sein.

Es wurde festgestellt, dass eine spezielle Injektion bei bis dahin in der Zyklusruhe befindlichen Tieren eine terminliche Steuerung des beginnenden Follikelwachstums an den Eierstöcken bewirkt. Hierdurch wird im Sinne des Zyklusstartes die Voraussetzung für eine Synchronisation der Follikelreifung, der Sekretion der Brunsthormone und der eintretenden Östren bei den behandelten Tieren geschaffen. Die Wirkung basiert sowohl auf einer Anregung von Funktionen des Hypophysen-Vorderlappens als auch der Ovarien, wobei aus dem ruhenden Pool der Follikelpopulation eine in physiologischen Grenzen oder darüberliegende Anzahl von Oozyten innerhalb einer kurzen Zeitspanne gleichzeitig in die Wachstumsphase eintritt. Dieser Effekt wird für die nachfolgende Belegung, vornehmlich künstliche Besamung, genutzt.

Beim erfindungsgemäßen Verfahren werden Mengen von 5 µg bis 500 µg Wirkstoff je ml Injektionsflüssigkeit eingesetzt. Vorzugsweise betragen die Wirkstoffmengen 50 bis 300 µg und insbesondere 100 bis 200 µg je ml Injektionsflüssigkeit.

Eine geeignete Einsatzvariante sind wässrige Lösungen, die neben dem Wirkstoff weitere übliche Hilfsstoffe enthalten können.

Je Tier werden Mengen von 25 bis 500 µg vorzugsweise 50 bis 300 µg und insbesondere 100 bis 200 µg an Wirkstoffen bei jeder Injektion jedem Tier verabreicht.

Die Applikation der Injektionslösung erfolgt in einer Zeitspanne von 0 bis 96 Stunden, vorzugsweise 12 bis 48 Stunden und insbesondere 24 Stunden nach Beendigung einer vorherigen Zyklusruhe bzw. -hemmung oder einer biotechnischen Vorbehandlung.

Geeignete Applikationen beim erfindungsgemäßen Verfahren der Wirkstoffmittel und seiner Modifizierungen bei Zuchttieren sind über implantierte osmotische Pumpen, über eine Mehrfachinjektion, als Depotpräparat bzw. als Infusion mit Depotwirkung möglich. Bei der Anwendung als Depotpräparat ergibt sich der Vorteil, dass mit einmaliger Gabe des Präparates die Wirkstoffmenge für die Auslösung des gewünschten Zyklusstartes den Tieren zugeführt wurde. Das trägt sehr zum Stressabbau bei.

Mit dem erfindungsgemäßen Verfahren steht ein praktikables Verfahren zum Zyklusstart von gruppenweise gehaltenen Zuchttieren zur Verfügung. Vorzügliche Ergebnisse wurden dabei bei gruppenweise gehaltenen Zuchtsauen erhalten.

Die Erfindung soll nachfolgend an einem Ausführungsbeispiel näher erläutert werden.

Nach Beendigung der vierwöchigen Säugezeit werden bei Zuchtsauen die Ferkel abgetrennt. 24 Stunden nach der Abtrennung wird den Zuchtsauen je Tier eine Injektionsflüssigkeit, die 125 µg Wirkstoff enthält, zum Zyklusstart verabreicht.

Sollte eine duldungsorientierte Besamung vorgesehen sein, so erfolgt eine Kontrolle der durch die angeregten Follikel und deren Östrogenproduktion eintretenden Östren. es erfolgen ein bis drei Inseminationen je Östrus.

Sollte eine terminorientierte Besamung vorgesehen sein, so erfolgt in einem Abstand von 72 h nach der Injektion, die das Follikelwachstum stimuliert, eine die Ovulation auslösende Injektion mit einer Injektionslösung, die als Wirkstoff D-Phe⁶-Gonadorelin enthält.

Die Besamung erfolgt terminorientiert. Die erste Besamung wird 24 bis 26 Stunden nach der die Ovulation auslösenden Injektion durchgeführt und die zweite Insemination erfolgt spätestens weitere 18 Stunden danach.

## Patentansprüche

1. Verfahren zum Zyklusstart bei Sauen nach vorangegangener Zyklusruhe, **dadurch gekennzeichnet, dass** den Tieren eine Injektionslösung injiziert wird, die als Wirkstoff synthetisch hergestellte FSH-Releasinghormone mit bekannter Sequenz, bestehend aus 10 Aminosäuren (Dekapeptid) enthält.

2. Verfahren nach Anspruch 1, **dadurch gekennzeichnet, dass** das FSH-Releasinghormon die Aminosäuresequenz Pyr-His-Trp-Ser-His-Asp-Trp-Lys-Pro-Gly-NH₂ besitzt.

3. Verfahren nach Anspruch 1 und 2, **dadurch gekennzeichnet, dass** die Aminosäuresequenz durch Anfügen weiterer Bestandteile modifiziert ist.

4. Verfahren nach mindestens einem der vorhergehenden Ansprüche, **dadurch gekennzeichnet, dass** der Wirkstoff 0 bis 96 Stunden, vorzugsweise 12 bis 48 Stunden und insbesondere 24 Stunden nach Beendigung einer vorherigen Zyklusruhe bzw. -hemmung oder einer biotechnischen Vorbehandlung injiziert wird.

5. Verfahren nach mindestens einem der vorhergehenden Ansprüche, **dadurch gekennzeichnet, dass** Injektionslösungen mit einem Gehalt von 5 bis 500 µg, vorzugsweise 50 bis 300 µg und insbesondere 100 bis 200 µg Wirkstoff je ml Injektionslösung eingesetzt werden.

6. Verfahren nach mindestens einem der vorhergehenden Ansprüche, **dadurch gekennzeichnet, dass** die verabreichte Injektionslösung je Einzeltier 25 bis 500 µg, vorzugsweise 50 bis 300 µg und insbesondere 100 bis 200 µg an Wirkstoff enthält.

7. Verfahren nach mindestens einem der vorhergehenden Ansprüche, **dadurch gekennzeichnet, dass** in einem Abstand von 72 h nach der Injektion für den Zyklusstart eine die Ovulation auslösende Injektion mit dem Wirkstoff D-Phe⁶-Gonadorelin erfolgt.

## Claims

1. Method for inducing onset of the reproductive cycle of a sow after a preceding anestrus, **characterized in that** the animals are injected with an injection solution that contains as an active substance a synthetically produced FSH-releasing hormone with a known sequence consisting of 10 amino acids (decapeptide).

2. Method according to claim 1,
**characterized in that** the FSH-releasing hormone has the amino acid sequence Pyr-His-Trp-Ser His-Asp-Trp-Lys-Pro-Gly-NH₂.

3. Method according to claim 1 or 2,
**characterized in that** the amino acid sequence is modified by the addition of further components.

4. Method according to at least one of the preceding claims,
**characterized in that** the active substance is injected 0 to 96 hours, preferably 12 to 48 hours, and, more particularly, 24 hours after the end of a preceding anaestrus or inhibition or a biotechnical pretreatment.

5. Method according to at least one of the preceding claims,
**characterized in that** the injection solution with a content from 5 to 500 µg, preferably 50 to 300 µg and, more particularly, 100 to 200 µg of active substance per milliliter of injection solution are used.

6. Method according to at least one of the preceding claims,
**characterized in that** the injection solution administered per individual animal contains 25 to 500 µg, preferably 50 to 300 µg and, more particularly, 100 to 200 µg of active substance.

7. Method according to at least one of the preceding claims,
**characterized in that** at an interval of 72 hours after the injection for the cycle induction, an ovulation-triggering injection takes place with the active substance D-Phe⁶-Gonadorelin.

## Revendications

1. Procédé pour induire le début de cycle chez les truies après un arrêt préalable du cycle,
**caractérisé en ce que** l'on injecte aux animaux une solution injectable qui contient en tant que principe actif des hormones libératrices de FSH avec une séquence connue, préparées synthétiquement, consistant en 10 acides aminés (décapeptide).

2. Procédé selon la revendication 1,
**caractérisé en ce que** l'hormone libératrice de FSH possède la séquence d'acides aminés Pyr-His-Trp-Ser-His-Asp-Trp-Lys-Pro-Gly-NH₂.

3. Procédé selon la revendication 1 et 2,
**caractérisé en ce que** la séquence d'acides aminés est modifiée par addition d'autres composants.

4. Procédé selon au moins l'une des revendications précédentes,
**caractérisé en ce que** la substance active est injectée de 0 à 96 heures, de préférence de 12 à 48 heures et en particulier 24 heures suivant la fin d'un arrêt ou une inhibition de cycle préalable ou d'un prétraitement biotechnique.

5. Procédé selon au moins l'une des revendications précédentes,
**caractérisé en ce que** l'on emploie des solutions injectables ayant une teneur de 5 à 500 µg, de préférence de 50 à 300 µg et en particulier de 100 à 200 µg de principe actif par ml de solution injectable.

6. Procédé selon au moins l'une des revendications précédentes,
**caractérisé en ce que** la solution injectable administrée par animal, contient 25 à 500 µg, de préférence 50 à 300 µg et en particulier 100 à 200 µg de principe actif.

7. Procédé selon au moins l'une des revendications précédentes,
**caractérisé en ce que** dans un laps de temps de 72 heures après l'injection pour induire le début de cycle, on effectue une injection déclenchant l'ovulation avec le principe actif D-Phe⁶-Gonadorelin.
